Europäisches Patentamt

European Patent Office

Office européen des brevets

(19)

(11) Publication number: **0 278 164**
**A1**

# (12) EUROPEAN PATENT APPLICATION

(21) Application number: **87310735.3**

(22) Date of filing: **07.12.87**

(51) Int. Cl.⁴: **G01N 33/84**

(30) Priority: **28.01.87 US 7718**

(43) Date of publication of application:
**17.08.88 Bulletin 88/33**

(84) Designated Contracting States:
**BE CH DE ES FR GB IT LI NL SE**

(71) Applicant: **TECHNICON INSTRUMENTS**
**CORPORATION**
**511 Benedict Avenue**
**Tarrytown, New York 10591(US)**

(72) Inventor: **Rosenfeld, Henry J.**
**250 Ridgedale Avenue Unit A-4**
**Florham Park New Jersey 07932(US)**

(74) Representative: **Wain, Christopher Paul et al**
**A.A. THORNTON & CO. Northumberland**
**House 303-306 High Holborn**
**London WC1V 7LE(GB)**

(54) **Reagent and method for assay of serum chloride.**

(57) The direct, quantitative determination of chloride in serum is effected by contacting the serum with a reagent comprising ferric salt (e.g. ferric nitrate) and an acid, e.g. a sulfonic acid, to form an iron-chloride complex, and spectrophotometrically measuring the quantity of iron-chloride complex.

EP 0 278 164 A1

# REAGENT AND METHOD FOR ASSAY OF SERUM CHLORIDE

This invention relates to a reagent and method for the direct quantitative determination of chloride in blood serum.

West et al., Anal. Chem., volume 28, pp. 1834-1838 (1956), describe direct spectrophotometric determination of small amounts of chloride using iron (III) perchlorate in perchloric acid. West et al. recognized that chloro complexes of iron (III) exhibit an intense absorption band in the vicinity of 340 nm and thus lend themselves to quantitative measurement.

Fingerhut, Clinica Chimica Acta, volume 41, pp. 247-253 (1972), describes an automated method for dialyzed serum chloride determination for use with the Technicon AutoAnalyzer II system based on the reaction between ferric perchlorate and chloride ion in dilute perchloric acid. The method eliminated mercury pollution associated with an automated method widely used at that time.

Leon et al., Clin. Chem., volume 20, number 7, p. 872 (1974) describe a method similar to the method described by Fingerhut with the improvement that it was used on the Technicon SMA 12/60.

Law et al., United States Patent No. 4,278,440, describe a reagent useful for determining chloride in undialyzed blood serum. The reagent comprises perchloric acid, ferric perchlorate, and nonionic surfactant, and forms yellow chloro complex of iron (III) with serum chloride. Use of the surfactant prevents protein precipitation. The surfactant is present in an amount from about 0.1 to about 20% by weight.

We have now devised a reagent and method for the direct, quantitative determination of chloride in serum, which reagent and method do not require mercury or surfactant to prevent protein precipitation.

According to the present invention, a reagent for the direct quantitative determination of chloride in undialysed serum comprises a ferric salt and a suitable acid.

One embodiment of method of the invention comprises the steps of

(a) mixing said serum with a first reagent comprising a suitable acid and measuring the absorbance of the mixture to obtain a sample blank;

(b) mixing the serum-first reagent mixture with a second reagent comprising a suitable amount of a ferric salt and a suitable acid, thereby forming an iron-chloride complex solution; and

(c) spectrophotometrically measuring the absorbance of the iron-chloride complex solution.

We have found that the chloride can be determined quantitatively in dialysed or undialysed blood serum using reagents and methods of this invention which promote a reaction between chloride and ferric ions. As hereinbefore indicated, the prior art describes the use of perchloric acid, ferric perchlorate, and nonionic surfactant to determine small amounts of chloride, by measuring absorbed light of yellow chloro complexes of iron (III). Because perchloric aicd is a good serum protein precipitant, and because protein precipitation interferes with the quantitative measurement of absorbed light of the yellow chloro complexes of iron (III), nonionic surfactant is frequently used to suppress and/or eliminate blood protein precipitation. In the present invention, a suitable ferric salt and a suitable acid e.g. a sulfonic acid, are used to form the yellow chloro-iron (III) complex, and there is no necessity to employ surfactants to suppress and/or eliminate blood protein precipitation. Also, no separation process such as dialysis is needed for separating serum proteins. Furthermore, the present invention is a non-mercurimetric chloride assay compatible with the Technicon Chem 1 System, which does not allow for dialysis. The invention is also compatible with diagnostic procedures such as those which utilize the Gilford Spectrophotometer (Gilford), the Cobas Bio (Roche Diagnostics), or similar instruments.

When the Technicon Chem 1 System is used, preferred reagents of the invention further comprise an amount of surfactant sufficient to improve reagent flow properties, e.g. about 0.1% by weight. The level of surfactant present in the preferred embodiment is not sufficient to suppress and/or eliminate blood protein precipitation. The presence of sulfonic acids eliminates the need for high amounts of surfactants.

Although ferric nitrate is preferred in the reagent of the present invention, other suitable ferric salts which form the detectable iron-chloride complex may be used. Suitable ferric salts are those which have solubility in the reagent, do not exhibit an intense color when dissolved in the acid, and do not precipitate serum proteins. For example, ferric perchlorate is not suitable because of serum protein precipitation, while reagents having ferric sulfate or ferric ammonium citrate are too highly coloured.

Many acids may be used in the invention to obviate the need for large quantites of surfactants. Sulfonic acids are preferred, particularly sulfonic acids having the general formula $R-SO_3H$, wherein R is an alkyl group. R preferably has from 1 to 6 carbon atoms, more preferably from 1 to 4 carbon atoms. Protein precipitation tends to occur when R has more than 6 carbon atoms. In particular, methane-, ethane-, and amido-sulfonic acids are the preferred sulfonic acids.

Other acids which can be used in the present invention without the need for sufactant are phosphoric acid, fluoboric acid, dichloroacetic acid, trifluoroacetic acid, fluosilicic acid, and hexafluorophosphoric acid. Acids which cannot be used because they require the presence of surfactant to prevent protein precipitation are nitric, trichloroacetic, p-toluenesulfonic and methanedisulfonic acid.

Preferred reagents of the invention comprise a concentration of ferric nitrate ($9H_2O$) preferably from about 5 grams per liter to about 500 grams per liter, more preferably about 50 grams per liter. Preferred amounts of other suitable ferric salts are such as to provide a concentration of ferric ion equivalent to that concentration of ferric ion provided by the preferred amounts of ferric nitrate; i.e. preferably from about 0.0124 mol/liter to about 1.24 mol/liter, more preferably about 0.124 mol/liter.

Preferred reagents of the invention also comprise a concentration of suitable acid from about 0.04N to about 4N, preferably about 0.4N.

Reagents having amounts of ferric salt substantially less than 0.124 mol per liter have less reaction sensitivity, i.e. the amount of absorbance per unit of chloride added. However, such reagents are still useful in the invention for determining serum chloride. Reagents having amounts of ferric salt substantially greater than 0.124 mol per liter have higher reaction sensitivity. However, use of high amounts of ferric salt increases the absorbance of the reagent alone during sample blanking, which can compromise the accuracy of the assay. To maintain assay accuracy, high amounts of ferric salt must be accompanied by high amounts of acids to provide a "bleaching" effect of the color due to ferric salt. A disadvantage to having such high amounts of acids and ferric salts is that they cause protein precipitation. Therefore, high levels of ferric salt, necessarily accompanied by high levels of acid, require that the serum be dialyzed or else that surfactant in amounts significantly greater than about 0.1% be used.

A preferred method of the invention for direct quantitative determination of dialyzed or undialyzed serum chloride according to the invention comprises:

(a) mixing said serum with a first reagent comprising a suitable acid and measuring absorbance of the mixture to obtain a sample blank;

(b) mixing the serum-first reagent mixture with a second reagent comprising ferric salt and a suitable acid, thereby forming an iron-chloride complex solution; and

(c) measuring the absorbance of the iron-chloride complex solution. For serum chloride determination using the Technicon Chem 1 system, incubation of the serum-first reagent mixture for several minutes is required in order to measure absorbance of the mixture and obtain the sample blanks. Mixing step (b) is preferably carried out for about 0.5 minutes. Thereafter, measuring step (c) is performed. The measuring step is preferably carried out at 340 nm.

For serum chloride determination employing the Technicon Chem 1 System, both first reagent and second reagent preferably further comprise a surfactant solely to improve reagent flow properties.

In order that the invention may be more fully understood, the following Example is given by way of illustration only.

Example:

Serum chloride determination using Techicon Chem 1 System

A first reagent is formed which contains a small amount of ethanesulfonic acid (0.03N) to provide proper sample blanking. Additionally, 0.1% Triton X-100 is included in this first reagent to provide good reagent flow properties on Technicon Chem 1 System.

A second reagent contains 50 grams ferric nitrate (9 $H_2O$) per liter as the source of iron (III) for complexation with $Cl^-$. Additionally, ethanesulfonic acid (0.4N) is included to suppress the reagent blank so that the iron-chloride complex can be detected at 340 nm. In addition, 0.1% Triton X-100 is included in the second reagent to provide good reagent flow properties on Technicon Chem 1 System.

One µl serum sample is mixed with 7 µl first reagent. After incubation for 5 minutes at a constant temperature of between about 30 and about 37°C, absorbance at 340 nm is measured (1.0 mm path length) to obtain the sample blank. 7 µl of second reagent is combined with the serum sample-first reagent mixture, and incubation is continued.

Although the endpoint absorbance is stable for at least 8 minutes, certain icteric samples exhibit significant interference if the final reaction is allowed to proceed beyond 1 minute. Thus it is advisable to take the 0.5 minute absorbance for all samples (1.5 mm pathlength). A serum-based calibrator is used. Also, strict thermal regulation is followed because absorbance by the iron-chloride complex is highly temperature sensitive.

When an instrument other than the Technicon Chem 1 is utilized, for example, the Cobas Bio or a Gilford spectrophotometer, a low level of surfactant for proper reagent flow properties is no longer necessary.

Manual assays can be conducted on the Gilford spectrophotometer equipped with a temperature-regulated flow cell. Since the flow cell has a relatively wide pathlength (1 cm), absorbance readings at 340 nm may be offscale. In this case, an offpeak wavelength can be employed, e.g. 360-380 nm, to reduce the readings.

The chloride reagent described herein is applicable to automated analysis on the Cobas Bio (Roche). Working parameters can be selected which produce a fairly narrow pathlength, thus permitting reading at 340 nm. Reaction readings taken at 0.5 mm. intervals are preferred.

## Claims

1. A reagent useful for the direct quantitative determination of chloride in undialyzed serum, said reagent comprising a ferric salt and an acid, characterised in that the amount of ferric salt and the nature and amount of acid are such that, in use of the reagent, they do not cause serum protein precipitation.

2. A reagent according to claim 1, further comprising a sufficient amount preferably about 0.1% by weight, of surfactant to maintain suitable reagent flow properties.

3. A reagent according to claim 1 or 2, wherein the ferric salt is ferric nitrate.

4. A reagent according to claim 1, 2 or 3, wherein the concentration of ferric salt is from about 0.0124 mol per liter to about 1.24 mol per liter, preferably about 0.124 mol per liter.

5. A reagent according to any of claims 1 to 4, wherein the concentration of suitable acid is from about 0.04N to about 4N, preferably about 0.4N.

6. A reagent according to any of claims 1 to 5, wherein the acid is selected from an aliphatic sulfonic acid, fluoboric acid, phosphoric acid, dichloroacetic acid, trifluoroacetic acid, fluosilicic acid, and hexafluorophoric acid.

7. A reagent according to claim 6, wherein the sulfonic acid has from 1 to 6 carbon atoms.

8. A reagent according to claim 7, wherein the sulfonic acid is methanesulfonic acid, ethansulfonic acid or amidosulfonic acid.

9. A method for direct quantitative determination of chloride in undialyzed serum, comprising the steps of:

(a) mixing said serum with a first reagent comprising a suitable acid and measuring the absorbance of the mixture to obtain a sample blank;

(b) mixing the serum-first reagent mixture with a second reagent comprising a suitable amount of a ferric salt and a suitable acid, thereby forming an iron-chloride complex solution; and

(c) spectrophotometrically measuring the absorbance of the iron-chloride complex solution.

10. A method according to claim 9, wherein either the first reagent or the second reagent further comprises a sufficient amount of surfactant to maintain suitable reagent flow properties.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A,D | US-A-4 278 440 (W-T- LAW et al.) * column 3, lines 11-29; claims 1, 8 * | 1,2,4,5 ,9,10 | G 01 N 33/84 |
| A | WO-A-8 302 670 (AMERICAN MONITOR CORP.) * abstract; claims 1-4 * | 1,6,9 | |
| A | US-A-3 447 904 (C.O. RUPE) * columns 7, 8; examples 5-9; claims 1, 2, 6 * | 1 | |
| A | DE-A-3 501 826 (EIKEN KAGAKU K.K.) * claims 1, 6 * | 1,6-9 | |

TECHNICAL FIELDS SEARCHED (Int. Cl.4)

G 01 N 33/00

The present search report has been drawn up for all claims

| Place of search | Date of completion of the search | Examiner |
|---|---|---|
| BERLIN | 28-04-1988 | DE KOK A.J. |

EPO FORM 1503 03.82 (P0401)